# EUROPEAN PATENT SPECIFICATION

(11) **EP 3 265 794 B1**
(45) Date of publication and mention of the grant of the patent: **16.08.2023**
(21) Application number: 16713588.8
(22) Date of filing: 26.02.2016
(51) Int. Cl.: G01N 27/18, G01N 33/18

(54) **COMPONENT AS WELL AS A METHOD FOR DETECTING THE PRESENCE OF ONE OR SEVERAL SOLUTES IN A LIQUID STREAM**
KOMPONENTE SOWIE VERFAHREN ZUR DETEKTION DER ANWESENHEIT EINER ODER MEHRERER GELÖSTER SUBSTANZEN IN EINEM FLÜSSIGKEITSSTROM
ÉLÉMENT ET PROCÉDÉ PERMETTANT DE DÉTECTER LA PRÉSENCE D'UN OU DE PLUSIEURS SOLUTÉS DANS UN FLUX LIQUIDE

(30) Priority: 03.03.2015 BE 201505111
(43) Date of publication of application: 10.01.2018
(73) Proprietor: Sercal Belgium BVBA, 2170 Merksem (BE)
(72) Inventor: HASPESLAGH, Harold, 8610 Kortemark (BE); WILLEMEN, Karl, 2170 Merksem (BE)
(74) Representative: Van hunsel, Lieven M.S.
(86) International application number: PCT/IB2016/051077
(87) International publication number: WO 2016/139563

(56) References cited:
- EP-A1- 2 081 009
- CH-A2- 705 686
- GB-A- 1 501 644
- US-A- 5 581 189

## Description

The present invention first of all relates to a component for detecting the presence of one or several solutes in a liquid stream.

More particularly, the invention relates to such a component which is mainly formed of a tube section or which is to be placed in a tube section whereby the aim is that the liquid stream flows through the tube section and whereby the component is provided with at least one detection sensor with a surface which can be put into contact with the liquid stream.

A component according to the invention may be typically suitable for example for the early detection of the presence of so-called hard water containing dissolved calcium carbonate (CaCO₃) and/or dissolved magnesium dioxide (MgO₂) in the pipes of a heating system in order to generate an alarm when there is a risk of scale occurring in the pipes of the heating system and in the heating system itself.

However, the invention is not limited to components with which calcium ions (Ca²⁺) and/or magnesium ions (Mg²⁺) dissolved in a water stream can be detected; components with which also other substances can be detected in all kinds of liquid streams are within the scope of the invention as well.

Heating systems are designed more and more compact and many components of such heating systems are increasingly more sensitive and not resistant to scale.

The warmest places in a heating system, such as the area of a heat exchanger or a boiler, are the most subject to scale.

Further, in the new central heating systems, more water is being used now than was previously the case, whereby in case of a small leak in the system, much water is often added to the pipes.

These are all factors which may contribute to the formation of scale.

For the protection of the heating system, it is therefore of prime importance that the presence of hard water in the pipes concerned is detected at an early stage.

US5581189 discloses a device for detecting dissolved solids in water. GB1501644 discloses a sensor for monitoring carbonate in water using a stripping gas. CH705686 discloses the use of precipitation as as aspect of solute monitoring.

According to the present state of the art, there are no simple means available to that end, and it is therefore an object of the invention to provide a solution.

To this end, the present invention concerns a component for detecting the presence of one or several solutes in a liquid stream as set forth in claim 1.

A major advantage of such a component according to the invention is that it has precipitation means with which the solute or solutes in the liquid stream to be detected will be precipitated faster on the surface of a detection sensor, such that the presence of the substance or substances to be detected in the liquid stream can be observed earlier, more particularly before the same precipitation or deposit takes place on parts of an installation to be protected.

Another major advantage of a component according to the invention is that it can be manufactured with relatively simple and inexpensive parts and can be made compact.

According to the invention, the component is provided with two sensors, namely a detection sensor and a reference sensor, both having a surface that can be put into contact with the liquid stream, whereby aforesaid precipitation means are provided for modifying the physical state of the liquid stream in the vicinity of the surface of the detection sensor so that a precipitation or deposit of the substance or substances to be detected on the surface of the sensor can be achieved or accelerated, i.e. in the presence of that particular substance or substances in the liquid stream, but whereby such precipitation means are not provided however to modify the physical state of the liquid stream in the vicinity of the surface of the reference sensor, whereby both sensors measure the above-mentioned detection parameter concerned and whereby the component is provided with a comparator circuit for comparing the measurements of the detection parameter concerned originating from the detection sensor with the measurements originating from the reference sensor in order to generate an input signal for the alarm means.

In short, the reference sensor is used for measurements of the detection parameter without any significant amount of substance to be detected having precipitated on its surface, whereas with the detection sensor just the opposite is intended, i.e. to make an easy detectable amount of the substance to be detected precipitate on its surface as quickly as possible, whereby the aim is of course to obtain a difference as large as possible between the measurements of the detection parameter at the reference sensor and at the detection sensor.

By making use of a comparator circuit, the measurements of the detection parameter at the detection sensor and the reference sensor respectively, can thus be compared to one another, and more specifically a measure can be obtained for the above-mentioned difference between the measurements.

A major advantage of such a component according to the invention is that a very stable detecting method is obtained which depends little or not on the circumstances in the liquid stream itself, such as for example typically the temperature of this liquid stream which may be higher one time compared to the other.

Indeed, both sensors, the detection sensor as well as the reference sensor, are exposed to the same conditions, so that by determining the difference between the value of the detection parameter at the detection sensor and the value of the detection parameter at the reference sensor, the influence of temporary circumstances in the liquid stream is eliminated from the measurements as if it were.

According to a possible embodiment of a component in accordance with the invention, in order to form the precipitation means, means are provided with which an electric and/or magnetic field can be created in the liquid stream.

Between the detection sensor and the reference sensor can be applied a DC voltage, for example, whereby the detection sensor forms the negative pole, for example, whereas the reference sensor serves as a positive pole.

Naturally, such a situation promotes the precipitation or deposit of positively charged ions in the liquid stream, such as for example calcium ions (Ca²⁺) or magnesium ions (Mg²⁺) in water, on the detection sensor, whereas the negative potential at the reference sensor inhibits said precipitation or deposit of positively charged ions in the liquid stream.

Of course, depending on the circumstances, the voltage can be reversed (for example for the precipitation of negatively charged ions) or its level can be adjusted, for example to enhance or reduce the effect.

In a similar way, applying a magnetic field may in some cases promote a precipitation or deposit on the surface of the detection sensor, for example when particles which are subject to magnetism are present in the liquid stream.

According to a preferred embodiment of a component in accordance with the invention, in order to form the precipitation means, a heating element is additionally or alternatively provided in the detection sensor, designed to locally heat the liquid stream around the detection sensor.

Naturally, said heating of the liquid stream in the vicinity of the detection sensor is again designed to accelerate or improve the precipitation or deposit of a solute in the liquid stream on the surface of the detection sensor.

This is for example typically the case when the aim is to detect the presence of calcium ions in a water flow, whereby a reduced solubility of CO₂ in a warmer liquid stream results in the precipitation of calcium carbonate.

In other cases, whereby other substances must be detected, it is not excluded according to the invention, however, to do just the opposite and provide a cooling at the detection sensor in order to promote the precipitation or deposit of the substance concerned to be detected on the surface of the detection sensor.

In a preferred embodiment of a component according to the invention, the detection sensor and a possible reference sensor are resistive temperature sensors, in particular temperature sensors known as Pt100.

As is known, Pt hereby stands for a platinum resistance sensor whereas 100 stands for an electrical resistance of the sensor amounting to 100 Ohm.

With such a resistance sensor, the electrical resistance increases as the temperature rises, whereby such an increase of the electrical resistance can be easily measured with a simple electrical circuit.

The detection sensor is hereby preferably a Pt100 temperature sensor, whereby this temperature sensor itself serves as a heating element to heat the liquid stream in the vicinity of the surface of the detection sensor.

In this way, the detection sensor not only serves to measure the detection parameter, but also as a precipitation means with which the precipitation of the substance or substances to be detected is promoted.

Naturally, it is not excluded according to the invention to use other temperature sensors, for example Ni100 (nickel), Pt100, Pt500 and the like, or even temperature sensors which are not based on the change of an electrical resistance as a function of the temperature.

The advantage of such embodiments of a component according to the invention whereby use is made of a Pt100 temperature sensor, is that these temperature sensors are made of a noble metal and therefore are little or not subject to corrosion.

A Pt100 temperature sensor also offers a great advantage in that it has a very large temperature range (typically between -200°C and 850°C).

Another advantage of the use of the above-mentioned type of temperature sensor is that precise measurements can be easily carried out with them in a dynamic manner.

With an aforesaid embodiment of a component according to the invention, the inner temperature in the detection sensor concerned and in the reference sensor is taken as the detection parameter.

As a matter of fact, a large difference in thermal conductivity can be observed with a detection sensor having a layer of material such as scale precipitated on its surface, as compared to the thermal conductivity that is observed with a reference sensor whose surface is free of such precipitated material.

Also, according to a preferred embodiment of a component according to the invention, the component is provided with a comparator circuit which also contains a pulse generator with which an electrical voltage pulse can be applied to the reference sensor as well as to the detection sensor and whereby the evolution of the inner temperatures, during the entire voltage pulse or during the transition phenomena associated with it, are measured in both sensors by the comparator circuit and whereby the difference between both measurements serves as a basis for generating an input signal for the alarm means.

It is understood that, as a result of the electrical current pulse, a certain heating of the sensors is obtained, whereby a reference sensor which is not covered by a layer of precipitated material cools faster in the liquid stream than a detection sensor which is covered by such a layer.

Also, the temperature in the detection sensor will increase faster, will rise to a higher value and will cool down slower than is the case in the reference sensor, at least when some material has precipitated on the detection sensor.

If the changes in the inner temperature occurring in the detection sensor and the reference sensor under the influence of the electrical pulse generated by the pulse generator differ from one another, this is a good indication for the presence of precipitated material on the surface of the detection sensor.

In a more general sense, the invention also concerns a method for detecting the presence of one or several solubles in a liquid as set forth in claim 9.

Such a method can also prove useful in chemical processes whereby the detection of certain harmful substances in a liquid stream is important.

In order to better explain the characteristics of the invention, the following preferred embodiments of a component according to the invention, as well as possible methods according to the invention, are described by way of example only without being limitative in any way, with reference to the accompanying figures, in which:
figure 1 schematically represents a first possible embodiment of a component according to the invention in a cross-sectional view;
figure 2 schematically represents the inner temperature curve in sensors of a component according to the invention as a function of time, as a result of a heating element being switched on and off;
figure 3 shows a view in perspective of another possible embodiment of a component according to the invention, in particular a component embedded in a metal (brass) housing;
figure 4 schematically represents an X-ray image through the head of a component according to the invention of a type as shown in figure 3;
figures 5 and 6 are views in perspective of yet another possible embodiment of a component according to the invention, this time embedded in a plastic housing, of the portion at the head of the component and of the entire component respectively, except for a portion of the cabling;
figure 7 is a block diagram illustrating the operation of a component according to the invention;
figure 8 represents a simple comparator circuit that can be used in a component according to the invention provided with resistance temperature sensors;
figure 9 schematically represents, in a similar way as in figure 2, the inner temperature curve in sensors of a component according to the invention as a function of time, as well as the voltage pattern at the output of a comparator circuit, as a result of the application of an electrical pulse;
figure 10 is a more realistic representation of the voltage pattern at the output of a comparator circuit as a result of the application of an electrical pulse; and,
figures 11 and 12 show the magnified portion of the graph from figure 10, indicated by F11/F12, when the temperature of the liquid stream amounts to 20°C and 60°C respectively.

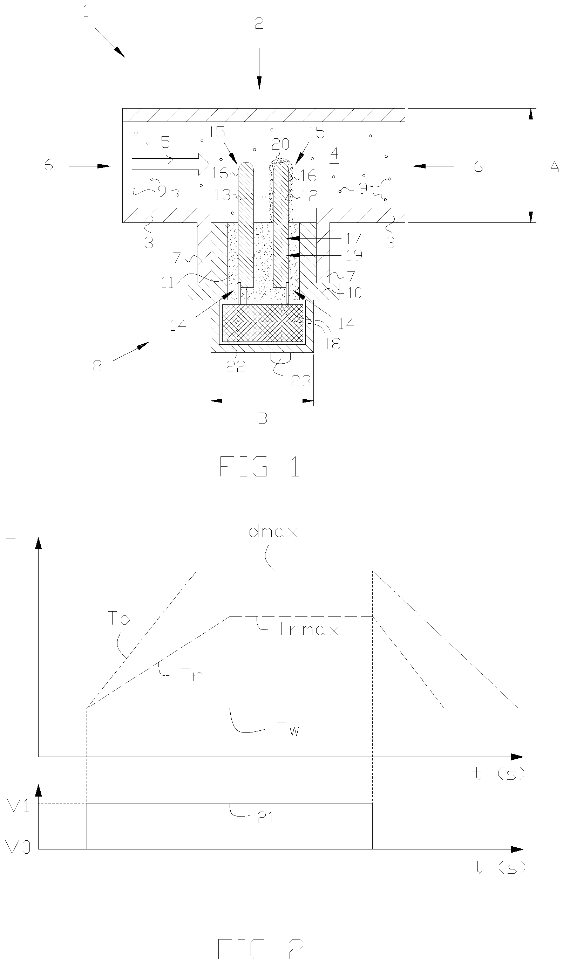

The component 1 according to the invention represented in figure 1 is designed for detecting the presence of one or several solutes in a liquid stream.

Typically, this substance may be for example dissolved calcium carbonate, but other solutes in a liquid stream such as calcium sulphate, barium sulphate, magnesium hydroxide, calcium phosphate, silicates and so on are not excluded from the invention either.

In practice, the liquid stream will be usually water, flowing for example in the pipes of a heating system, but the invention is equally applicable for detecting substances in flows of other liquids, such as for example liquids that are common in refrigeration systems and the like.

In the example shown in figure 1, the component 1 is mainly formed of a T-shaped tube section 2 which is made for example of a metal such as copper or brass or steel, but better still of a plastic or polymeric material which is preferably stable to hydrolysis, such as for example PPS or PA66 and the like.

The T-shaped tube section 2 is provided with two arms 3 extending in line with each other and which, for use in a heating system, preferably have a diameter A of 3/4" (0.75 inch or 19.05 mm), although other diameters are not excluded, of course.

The aim is for the liquid stream 4 to flow through these arms 3, which is schematically represented in figure 1 by the arrow 5.

The component 1 should thus be integrated in the pipe system to be protected, and to that end the tube section 2 on the far ends 6 of the arms 3 can be provided with external and internal threads and the like according to the known techniques.

The T-shaped tube section 2 also has a third arm 7 which in this case has a somewhat smaller diameter B, for example a diameter of 3/8" (0.375 inch or 9.78 mm), but other diameters B are also possible according to the invention, of course.

The essential portion 8 of the component 1, required for the detection of the solutes 9 in the liquid stream 4, is mounted In this third arm 7 of the T-shaped tube section.

The portion 8 includes a sleeve 10 which fits in the third arm 7.

The sleeve 10 is filled with an electrically insulating resin 11 in which two sensors 12 and 13 are embedded with their feet 14, more specifically a detection sensor 12 and a reference sensor 13.

Both sensors 12 and 13 further extend into the middle of the T-shaped tube section 2 and have a surface 16 on their heads 15 which can make contact with the liquid stream 4, at least in so far as the liquid stream 4 flows through the arms 3 of the tube section 2.

The portion 8 fills the third arm 7 and hermetically seals it from the liquid stream 4.

In the given example, the detection sensor 12 and the reference sensor 13 are carried out as resistive temperature sensors, more specifically temperature sensors known as Pt100-sensors provided on a ceramic (Al₂O₃) and over which an insulating and protective glazing has been provided.

In essence, such Pt100 temperature sensors form an electrical resistance whose magnitude rises as the temperature rises.

The detection sensor 12 is in this case a temperature sensor which also serves as a heating element 17 for heating the liquid stream 4 in the vicinity of the surface 16 of the detection sensor 12.

For this purpose, it is sufficient in this case to make an electrical current flow through the temperature sensor 12 via the connections 18 at the base 14 of the temperature sensor 12.

After all, in the electrical resistance of the temperature sensor 12, the electric energy of the electrical current is converted into heat.

In other embodiments, a separate heating element 17 may be provided, for example.

For, in the most general terms, the aim according to the invention is that the component 1 is provided with precipitation means 19 with which the physical state of the liquid stream 4 in the vicinity of the surface 16 of the detection sensor 12 can be altered, all this with the aim to effect or accelerate a precipitation or deposit of the substance or substances to be detected 9 on the surface 16 concerned of the detection sensor 12, i.e. in the presence of that particular substance or substances 9 in the liquid stream 4.

In an embodiment whereby the aim is to apply an electric field with the precipitation means 19 to make the substances 9 to be detected precipitate, it is not excluded according to the invention to apply a thin film of noble metal (e.g. Pt/Pd) on the glazing of the Pt100 temperature sensors.

In this manner, such a PT100 temperature sensor can also serve as an electrode for generating the electric field in the liquid stream 4.

By making use of a thin film of noble metal, the thin film will be less subject to chemical corrosion by the liquid stream 4.

In the case as discussed here, the precipitation means 19 are formed by the detection sensor 12 itself, in the sense that the detection sensor 12 can serve as a heating element 17, whereby the liquid stream 4 undergoes a physical state modification in the vicinity of the surface 16 of the detection sensor 12 which consists in that the liquid 4 is locally heated.

Assuming that in this case, for example calcium ions 9 need to be detected in a water stream 4, said heating of the water stream 4 in the vicinity of the surface 16 of the detection sensor 12 will indeed have as an effect that the calcium ions (Ca²⁺) will tend to precipitate on the surface 16 in the shape of a precipitation layer 20 formed of calcium carbonate (CaCO₃), which is the purpose according to the invention.

In order to have some effect as a precipitation means 19, it is of course required that an electrical current flows through the temperature sensor 12 for a considerable time, such that the temperature sensor 12 can serve long enough as a heating element 17 to effect the precipitation or deposit of the substances 9.

In the embodiment of a component 1 according to the invention as discussed here, as shown in figure 1, it is further intended that the reference sensor 13 should not be provided with said precipitation means 18 to alter the physical state of the liquid stream 4 in the vicinity of the surface 16 of the reference sensor 13, such that the reference sensor 13 can indeed be used as a "reference", more specifically a sensor 13 on which no precipitation layer 19 has been deposited.

That is why the temperature sensor 13 which serves as a reference sensor 13 is not used as a heating element 17 and thus is not flown through by an electrical current, or at least not long enough, so that a precipitation effect on the surface 16 thereof could not be induced in any way. Further, it is the aim according to the invention to measure a detection parameter with the sensors 12 and 13 which is influenced by the presence of a precipitated material 20 on the surface 16 of the detection sensor 12. In this case, the above-mentioned detection parameter is the inner temperature in the detection sensor 12 concerned, combined with the inner temperature measured in the reference sensor 13, which offers many advantages as already explained in the introduction.

The inner temperature in the sensors 12 and 13 is indeed influenced by a precipitation layer 20 being either or not present on the detection sensor 12, since during the internal heating of both sensors 12 and 13, the same amount of generated heat can escape more easily through the surface 16 of the reference sensor 13 which is cooled directly by the liquid stream 4 than through the surface 16 of the detection sensor 12 which is covered by a precipitation layer 20 forming a heat-insulating layer.

The temporary internal heating of the sensors 12 and 13 can be easily achieved by sending an electrical pulse 21 through the resistance of the temperature sensors 12 and 13.

This results in an inner temperature curve T as is outlined in figure 2, whereby V0 and V1 represent the respective conditions whereby no electrical current passes through the sensors 12 and 13, i.e. when there is no internal heating on, and whereby an electrical current flows in the sensors 12 and 13, i.e. when internal heating occurs.

In the upper part of figure 2 it is clear to see that, after the heating has been switched on, in the detection sensor 12 (which is covered with a precipitation layer 20), the inner temperature Td will rise faster above the water temperature Tw than the inner temperature Tr in the reference sensor 13, and that the inner temperature Td in the detection sensor 12 will rise to a maximum temperature Tdmax which is higher than the maximum inner temperature Trmax in the reference sensor 13.

Analogously, when the heating is switched off, the temperature Td in the detection sensor 12 will decrease slower from the Tdmax value to the ambient temperature Tw of the liquid stream 4 than is the case with the reference sensor 13.

This difference in behaviour while a heating element in the sensors 12 and 13 is temporarily switched on is used according to the invention to detect the presence of the precipitation layer 20.

To this end, electronics 22 are provided in the component 1 with which the necessary algorithms can be carried out in order to draw a correct conclusion regarding the detection of the presence of a precipitated layer 20 on the basis of measurements of the detection parameter(s).

The component 1 is further provided with alarm means 23 to generate an alarm in case a certain degree of modification of the detection parameter concerned is changed or exceeded so as to indicate that the presence of the particular substance or substances was detected.

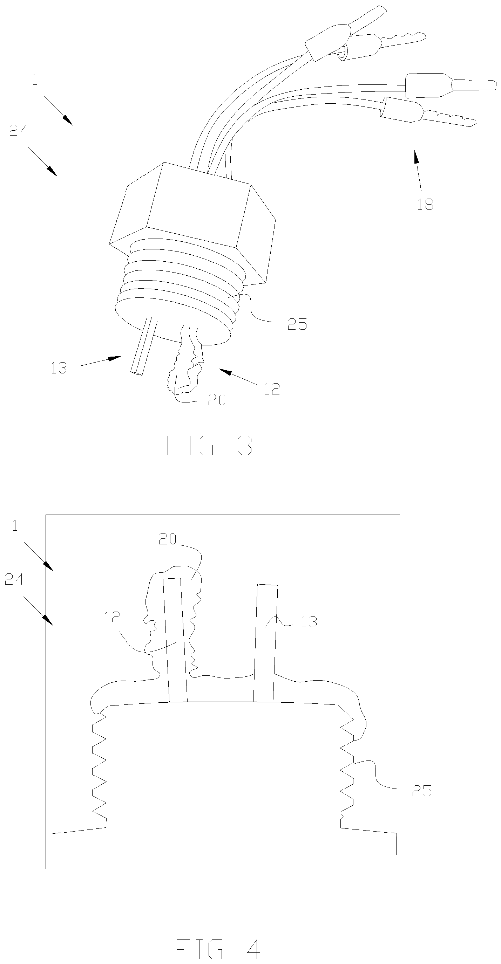

Figures 3 and 4 represent another possible embodiment of a component 1 according to the invention in a more realistic manner.

In this case, the component 1 itself does not form a tube section 2, but the component 1 is made rather as a kind of brass stop 24 with external thread 25 which can be screwed in an opening with inner thread of a tube section of for example a heating system, whereby the liquid stream 4 in this case flows through the latter tube section.

Again, a detection sensor 12 and reference sensor 13 are provided whose surface 16 on the head 15 thereof can be put into contact with the liquid stream 4.

Scale 20 on the detection sensor 12 is hereby clearly perceptible, which is shown in even more detail in the illustration of figure 4, which represents an X-ray scan of the same component 1 from figure 3.

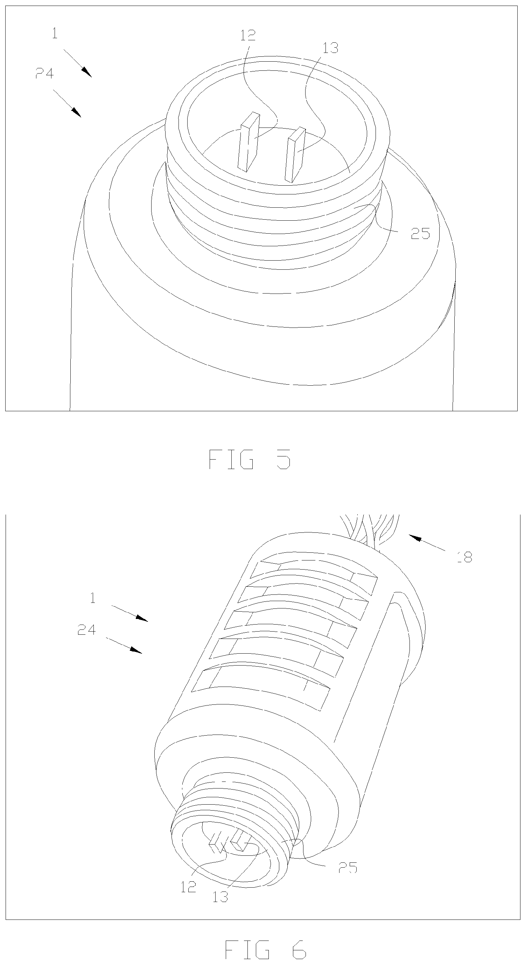

Figures 5 and 6 represent a component 1 according to the invention which is made entirely similar to a stop 24 as in the embodiment of figures 3 and 4, apart from the fact that this stop 24 is elongate and its housing is made of PA 66.

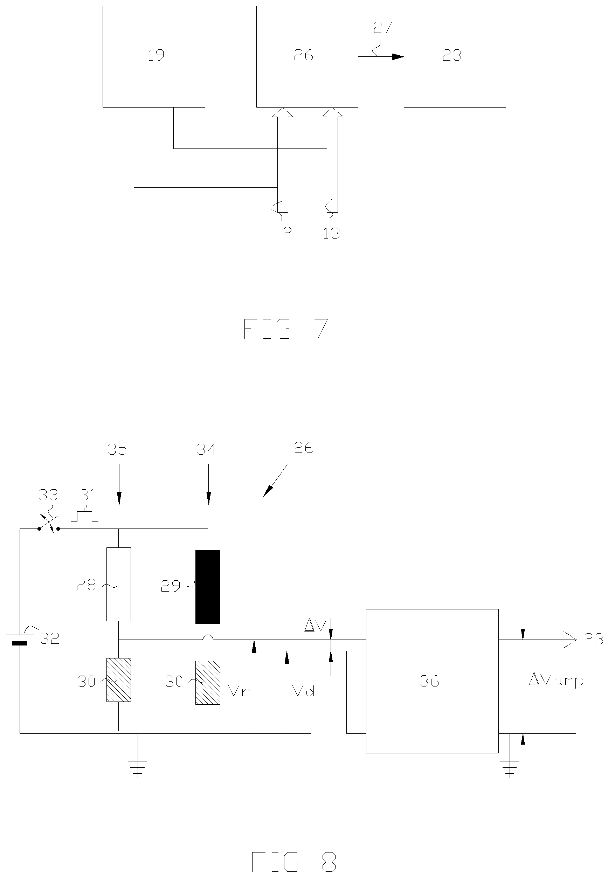

In figure 7, a method according to the invention is summarized once more in a block diagram.

In a first step, an attempt is made to obtain a difference in precipitation speed by means of precipitation means 19 as the substance 9 to be detected precipitates either on the detection sensor 12 or on the reference sensor 13.

These precipitation means 19 may aim a local heating, but they may just as well involve the application of a magnetic or electric field or even the injection of certain chemical substances at the detection sensor and the like.

As a result is obtained a difference in thickness of precipitated material 20 on the surfaces 16 of the sensors 12 and 13.

The sensors 12 and 13 each measure a detection parameter which is influenced by the thickness of the precipitation layer 20 on the sensor 12 or 13 concerned and thus, different measured values of this detection parameter are obtained at the detection sensor 12 and the reference sensor 13 respectively.

These measured values of the detection parameter coming from the detection sensor 12 and the reference sensor 13 are offered to a comparator circuit 26 which generates an input signal 27 for the alarm means 23.

Figure 8 shows with an electric circuit diagram how such a comparator circuit 26 can be easily obtained.

The comparator circuit 26 contains sensor resistors 28 and 29 representing the inner electrical resistances of the reference sensor 12 and the detection sensor 13 respectively, whereby these resistors 28 and 29 thus increase in value as the temperature rises.

A resistor 30 is connected in series to each sensor resistor 28 and 29, whereby each resistance 30 is equally large, for example 1 Ohm.

These resistors 30 are measuring resistors 30 whose value is in principle not variable as a function of the temperature, and they are intended for the actual measurement of the detection parameter.

An identical voltage pulse 31 is applied in parallel over each of the sensors 12 and 13 and their respective measuring resistors 30 by means of a direct current source 32 and a pulse generator 33.

In this manner is obtained a temporary heating of the sensors 12 and 13, whereby the detection sensor 12 acquires a higher temperature than the reference sensor 13 in the presence of a precipitation layer 20, and consequently also the sensor resistor 29 gets warmer than the sensor resistor 28, resulting in a higher electrical resistance value for the sensor resistor 28 compared to the resistance value of the sensor resistor 29.

Since both sensors 28 and 29 and their respective measuring resistors are subjected to an identical voltage pulse 31, less electrical current will flow in the branch 34 of the comparator circuit 26 in which the inner resistor 29 of the detection sensor 12 has been implemented than in the branch 35 of the other resistor 28.

This results in a voltage drop Vr over the measuring resistor 30 that is coupled to the reference sensor 13 which is higher than the voltage Vd over the measuring resistor 30 of the detection sensor 12.

Thus, we have a voltage difference ΔV = Vr - Vd which is a measure for the available precipitated material 20 on the surface 16 of the detection sensor 12.

However, this voltage difference ΔV is very small and is preferably amplified to a voltage difference ΔVamp by means of an amplifier 36 before being fed to the alarm means 23.

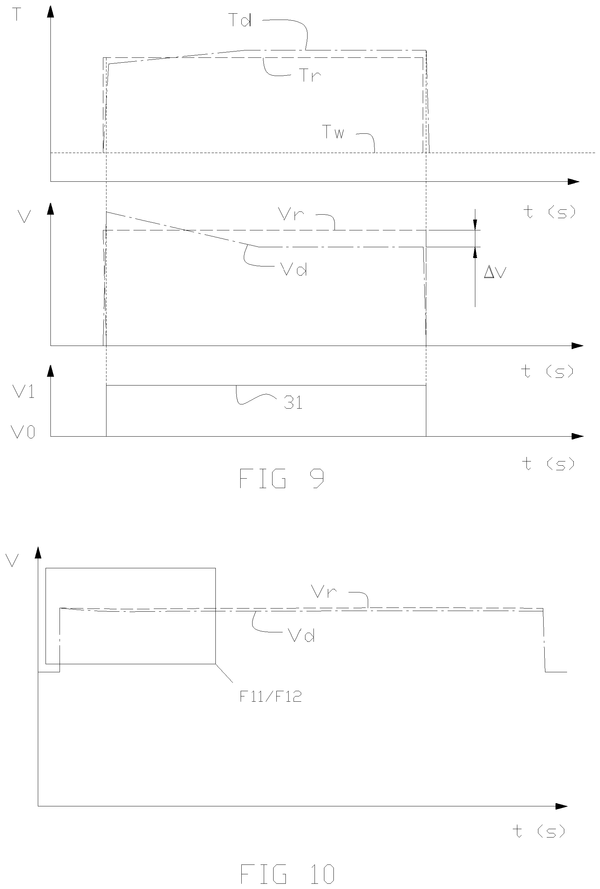

Everything is schematically summarized once more in figure 9, whereby the pulse tension 31 is represented as a function of time in the lower graph, the resulting tensions Vr, Vd and ΔV generated in the comparator circuit are represented as a function of time in the middle graph and the temperatures Td and Tr in the detection sensor 12 and the reference sensor 13 respectively are also represented as a function of time.

Figures 10 to 12 again represent the voltage pulse 31 together with the tensions Vr, Vd and ΔV measured in a real component 1 according to the invention in order to demonstrate that the measured voltage difference ΔV is very small.

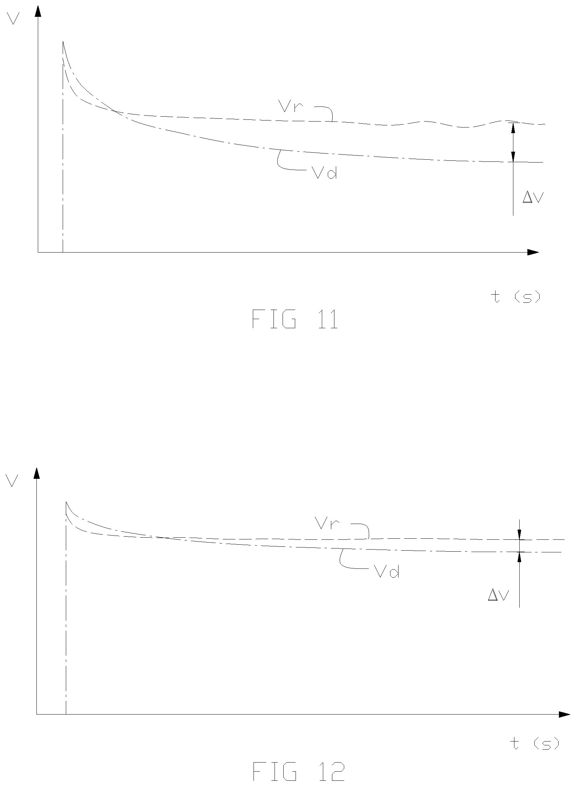

Figures 11 and 12 again represent the situation, but this time enlarged, for the situation in which the temperature Tw of the water stream amounts to 20°C and 60°C respectively.

This clearly shows that also the water temperature Tw has a certain influence on the realized voltage difference ΔV which is a measure for the thickness of the precipitation layer 20.

Nevertheless, the difference is not large. With a voltage pulse 31 of 12 V, ΔV = 0.25 V for example in the first case of figure 11, whereas ΔV - 0.20 V in the case of figure 12.

## Claims

1. Component (1) for detecting the presence of one or several solutes (9) in a liquid stream (4), which is mainly formed of a tube section (2) or which should be placed in a tube section through which the liquid stream (4) is to be applied and whereby the component (1) is provided with at least one detection sensor (12) having a surface (16) that can be put into contact with the liquid stream (4), whereby the component (1) is further provided with precipitation means (19) for changing the physical state of the liquid stream (4) in the vicinity of the surface (16) of the detection sensor (12) so that a precipitation or deposit of the substance or substances to be detected (9) on the surface (16) of the detection sensor (12) can be achieved or accelerated, i.e. in the presence of that particular substance or substances (9) in the liquid stream (4); in that a detection parameter (Td) which is influenced by the presence of precipitated material (20) on the surface (16) can be measured with the detection sensor (12); and in that the component (1) is provided with alarm means (23) for generating an alarm when a certain degree of modification of the detection parameter (Td) concerned is altered or exceeded to indicate that the presence of the substance or substances (9) concerned has been detected, **characterised in that** the component (1) additionally comprises a reference sensor (13), having a surface (16) that can be put into contact with the liquid stream (4), whereby aforesaid precipitation means (19) are not provided however to modify the physical state of the liquid stream (4) in the vicinity of the surface of the reference sensor (13) and whereby a detection parameter (Tr) which is not influenced by the presence of precipitation material on the detection sensor (12) can be measured with the reference sensor (13) and whereby the component (1) is provided with a comparator circuit (26) for comparing the measurements of the detection parameter (Td) concerned originating from the detection sensor (12) with the measurements (Tr) originating from the reference sensor (13) in order to generate an input signal for the alarm means (23).

2. Component (1) according to claim 1, **characterised in that** the substances (9) to be detected are dissolved calcium ions and **in that** the liquid stream (4) is a water stream.

3. Component (1) according to one or several of the preceding claims, **characterised in that**, in order to form the precipitation means (19), means are provided with which an electric and/or magnetic field can be generated in the liquid stream (4).

4. Component (1) according to one or several of the preceding claims, **characterised in that**, in order to form the precipitation means (19), a heating element (17) is provided in the detection sensor (12) which is designed to locally heat the liquid stream (4) around the detection sensor (12).

5. Component (1) according to one or several of the preceding claims, **characterised in that** the detection sensor (12) and the reference sensor (13) are resistive temperature sensors, more specifically temperature sensors known as Pt100-sensors, which stands for a platinum resistance sensor with a resistance of 100 Ohm and whereby the aforesaid detection parameter (Td,Tr) is the inner temperature in the detection sensor (12) and reference sensor (13) concerned.

6. Component (1) according to one or several of the preceding claims, **characterised in that** the detection sensor (12) is a Pt100 temperature sensor whereby this temperature sensor itself serves as a heating element (17) for heating the liquid stream (4) in the vicinity of the surface (16) of the detection sensor (12).

7. Component (1) according to claims 1 and 5, **characterised in that** the comparator circuit (26) also contains a pulse generator (33) with which an electrical voltage pulse (31) can be applied to the reference sensor (13) as well as to the detection sensor (12) and whereby the evolution of the inner temperatures (Td,Tr), during the entire voltage pulse (31) or during the transition phenomena associated with it, are measured in both sensors (12,13) by the comparator circuit (26) and whereby the difference (ΔV) between both measurements (Vr,Vd) serves as a basis for generating an input signal for the alarm means (23).

8. Component (1) according to one or several of the preceding claims, **characterised in that** the component (1) is made with a brass or plastic housing (2) with external thread (25) which can be screwed in a hole with inner thread of a conduit.

9. Method for detecting the presence of one or several solutes (9) in a liquid stream (4), whereby it consists in:
- providing a detection sensor (12) in the liquid stream (4);
- changing the physical state of the liquid stream (4) around the detection sensor (12) in order to effectuate or accelerate a precipitation or deposit of the substance or substances to be detected (9) on the detection sensor (12), provided the substance or substances (9) concerned is/are present in the liquid stream (4);
- measuring a detection parameter (Td) with the detection sensor (12), which parameter is influenced by the presence of precipitated material (20) on the detection sensor (12); and,
- when a certain degree of modification of the detection parameter (Td) concerned is altered or exceeded, generating an alarm to indicate that the presence of the substance or substances (9) concerned has been detected, **characterised in that** use is made of a reference sensor (13) in the liquid stream (4), configured to detect a detection parameter (Tr) which is not influenced by the presence of precipitation material on the detection sensor (12) whereby in the vicinity of the detection sensor (12), a physical state is modified in order to achieve or accelerate a precipitation or deposit of the substance or substances to be detected (9) on the detection sensor (12), but not at the reference sensor (13), and whereby in order to detect the presence of the substance or substances (9) in the liquid stream (4), the detection parameter (Td,Tr) concerned is measured in both sensors (12,13) and these measurements (Vr,Vd) are compared with one another.

10. Method according to claim 9, **characterised in that** the substances (9) to be detected in the liquid stream (4) are dissolved calcium ions and/or magnesium ions and **in that** the liquid stream (4) is a water stream.

11. Method according to claim 9 or 10, **characterised in that** the physical state which is modified around the detection sensor (12) is an increased temperature of the liquid stream (4) in the vicinity of the detection sensor (12).

12. Method according to one or several of claims 9 to 11, **characterised in that** the physical state which is modified around the detection sensor (12) is a modified electric or magnetic field.

13. Method according to one or several of claims 9 to 12, **characterised in that** the detection parameter (Td) which is measured by the detection sensor (12) in order to detect the presence of the substance or substances (9) is the heat transfer through or the inner temperature (Td) in the detection sensor (12).

## Patentansprüche

1. Komponente (1) zur Detektion des Vorhandenseins eines oder mehrerer gelöster Stoffe (9) in einem Flüssigkeitsstrom (4), die hauptsächlich aus einem Rohrabschnitt (2) gebildet ist oder die in einen Rohrabschnitt gelegt werden soll, durch den der Flüssigkeitsstrom (4) aufzubringen ist, und wobei die Komponente (1) mit mindestens einem Detektionssensor (12) versehen ist, der eine mit dem Flüssigkeitsstrom (4) in Kontakt bringbare Oberfläche (16) hat, wobei die Komponente (1) ferner mit Ausfällungsmitteln (19) zur Änderung des physikalischen Zustands des Flüssigkeitsstroms (4) in der Nähe der Oberfläche (16) des Detektionssensors (12) versehen ist, so dass eine Ausfällung oder eine Ablagerung des oder der zu detektierenden Stoffe (9) auf der Oberfläche (16) des Detektionssensors (12) bewirkt oder beschleunigt werden kann, d. h. in Gegenwart dieses bestimmten Stoffes oder dieser bestimmten Stoffen (9) in dem Flüssigkeitsstrom (4); wobei mit dem Detektionssensor (12) ein Detektionsparameter (Td) gemessen werden kann, der durch die Anwesenheit von gefälltem Material (20) auf der Oberfläche (16) beeinflusst wird; und wobei die Komponente (1) mit Alarmmitteln (23) zur Erzeugung eines Alarms versehen ist, wenn eine bestimmte Änderung des betreffenden Detektionsparameters (Td) geändert oder überschritten wird, um anzuzeigen, dass das Vorhandensein des betreffenden Stoffes oder der betreffenden Stoffe (9) festgestellt wurde, **dadurch gekennzeichnet, dass** die Komponente (1) zusätzlich einen Referenzsensor (13) umfasst, der eine mit dem Flüssigkeitsstrom (4) in Kontakt bringbare Oberfläche (16) hat, wobei die oben genannten Ausfällungsmittel (19) jedoch nicht vorgesehen sind, den physikalischen Zustand des Flüssigkeitsstroms (4) in der Nähe der Oberfläche des Referenzsensors (13) zu verändern, und wobei mit dem Referenzsensor (13) ein Detektionsparameter (Tr) gemessen werden kann, der durch die Anwesenheit von Ausfällungsmaterial auf dem Detektionssensor (12) nicht beeinflusst wird, und wobei die Komponente (1) mit einer Komparatorschaltung (26) versehen ist, um die Messungen des betreffenden Detektionsparameters (Td) von dem Detektionssensor (12) mit den Messungen (Tr) von dem Referenzsensor (13) zu vergleichen, um ein Eingangssignal für das Alarmmittel (23) zu erzeugen.

2. Komponente (1) nach Anspruch 1, **dadurch gekennzeichnet, dass** die zu detektierenden Stoffe (9) gelöste Calciumionen sind, und dass der Flüssigkeitsstrom (4) ein Wasserstrom ist.

3. Komponente (1) nach einem oder mehreren der vorhergehenden Ansprüche, **dadurch gekennzeichnet, dass** zur Ausbildung der Ausfällungsmittel (19) Mittel vorgesehen sind, mit denen in dem Flüssigkeitsstrom (4) ein elektrisches und/oder magnetisches Feld erzeugt werden kann.

4. Komponente (1) nach einem oder mehreren der vorhergehenden Ansprüche, **dadurch gekennzeichnet, dass** zur Ausbildung der Ausfällungsmittel (19) ein Heizelement (17) in dem Detektionssensor (12) vorgesehen ist, welches dazu ausgelegt ist, den Flüssigkeitsstrom (4) um den Detektionssensor (12) herum lokal zu erwärmen.

5. Komponente (1) nach einem oder mehreren der vorhergehenden Ansprüche, **dadurch gekennzeichnet, dass** der Detektionssensor (12) und der Referenzsensor (13) resistive Temperatursensoren sind, insbesondere Temperatursensoren, die als Pt100-Sensoren genannt werden, die für einen Platin-Widerstandssensor mit einem Widerstand von 100 Ohm stehen, und wobei der oben genannte Detektionsparameter (Td, Tr) die Innentemperatur in den betreffenden Detektionssensor (12) und Referenzsensor (13) ist.

6. Komponente (1) nach einem oder mehreren der vorhergehenden Ansprüche, **dadurch gekennzeichnet, dass** der Detektionssensor (12) ein Pt100-Temperatursensor ist, wobei dieser Temperatursensor selbst als Heizelement (17) zur Erwärmung des Flüssigkeitsstroms (4) in der Nähe der Oberfläche (16) des Detektionssensors (12) dient.

7. Komponente (1) nach den Ansprüchen 1 und 5, **dadurch gekennzeichnet, dass** die Komparatorschaltung (26) auch einen Impulsgeber (33) enthält, mit dem ein elektrischer Spannungsimpuls (31) auf den Referenzsensor (13) sowie auf den Detektionssensor (12) aufgebracht werden kann, und wobei die Entwicklung der inneren Temperaturen (Td,Tr) während des gesamten Spannungsimpulses (31) oder während der damit verbundenen Übergangsphänomene in beiden Sensoren (12,13) durch die Komparatorschaltung (26) gemessen werden, und wobei die Differenz (ΔV) zwischen beiden Messungen (Vr,Vd) als Grundlage zu der Erzeugung eines Eingangssignals für das Alarmmittel (23) dient.

8. Komponente (1) nach einem oder mehreren der vorhergehenden Ansprüche, **dadurch gekennzeichnet, dass** die Komponente (1) mit einem Messing- oder Kunststoffgehäuse (2) versehen ist, das ein Außengewinde (25) umfasst, das in eine Bohrung, die ein Innengewinde einer Leitung umfasst, einschraubbar ist.

9. Verfahren zur Detektion des Vorhandenseins eines oder mehrerer gelöster Stoffe (9) in einem Flüssigkeitsstrom (4), wobei es darin besteht:
- das Bereitstellen eines Detektionssensors (12) in dem Flüssigkeitsstrom (4);
- die Änderung des physikalischen Zustands des Flüssigkeitsstroms (4) um den Detektionssensor (12) herum, um eine Ausfällung oder eine Ablagerung des zu detektierenden Stoffes (9) auf dem Detektionssensor (12) zu bewirken oder zu beschleunigen, sofern der betreffende Stoff oder die betreffenden Stoffe (9) in dem Flüssigkeitsstrom (4) vorhanden ist/sind,
- die Messung eines Detektionsparameters (Td) mit dem Detektionssensor (12), dessen Parameter durch das Vorhandensein von gefälltem Material (20) auf dem Detektionssensor (12) beeinflusst wird; und
- wenn ein gewisses Maß an Änderung des betreffenden Detektionsparameters (Td) verändert oder überschritten wird, die Erzeugung eines Alarms, der anzeigt, dass das Vorhandensein des betreffenden Stoffes oder der betreffenden Stoffe (9) erkannt wurde, **dadurch gekennzeichnet, dass** in dem Flüssigkeitsstrom (4) ein Referenzsensor (13) verwendet wird, der zur Detektion eines Detektionsparameters (Tr) konfiguriert ist, der nicht durch das Vorhandensein von Ausfällungsmaterial auf dem Detektionssensor (12) beeinflusst wird, wobei in der Nähe des Detektionssensors (12) ein physikalischer Zustand geändert wird, um eine Ausfällung oder eine Ablagerung des zu detektierenden Stoffes oder der zu detektierenden Stoffe auf dem Detektionssensor (12), jedoch nicht auf dem Referenzsensor (13), zu bewirken oder zu beschleunigen (9), und wobei zur Detektion der Anwesenheit des Stoffes oder der Stoffe (9) in dem Flüssigkeitsstrom (4) der betreffende Detektionsparameter (Td, Tr) in beiden Sensoren (12,13) gemessen wird, und diese Messungen (Vr, Vd) miteinander verglichen werden.

10. Verfahren nach Anspruch 9, **dadurch gekennzeichnet, dass** die in dem Flüssigkeitsstrom (4) zu detektierenden Stoffe (9) gelöste Kalziumionen und/oder Magnesiumionen sind, und dass der Flüssigkeitsstrom (4) ein Wasserstrom ist.

11. Verfahren nach Anspruch 9 oder 10, **dadurch gekennzeichnet, dass** der um den Detektionssensor (12) herum veränderte physikalische Zustand eine erhöhte Temperatur des Flüssigkeitsstroms (4) in der Nähe des Detektionssensors (12) ist.

12. Verfahren nach einem oder mehreren der Ansprüche 9 bis 11, **dadurch gekennzeichnet, dass** der um den Detektionssensor (12) herum modifizierte physikalische Zustand ein modifiziertes elektrisches oder magnetisches Feld ist.

13. Verfahren nach einem oder mehreren der Ansprüche 9 bis 12, **dadurch gekennzeichnet, dass** der Detektionsparameter (Td), der vom Detektionssensor (12) zur Detektion des Vorhandenseins des Stoffes oder der Stoffe (9) gemessen wird, der Wärmeübergang durch den oder die Innentemperatur (Td) in dem Detektionssensor (12) ist.

## Revendications

1. Composant (1) destiné à détecter la présence d'un ou de plusieurs éléments dissous (9) dans un courant de liquide (4), qui est réalisé principalement à partir d'un tronçon tubulaire (2) ou qui doit venir se placer dans un tronçon tubulaire à travers lequel le courant de liquide (4) doit être appliqué ; et dans lequel le composant (1) est muni d'au moins un capteur de détection (12) qui possède une surface (16) qui peut être mise en contact avec le courant de liquide (4) ; dans lequel le composant (1) est en outre muni de moyens de précipitation (19) qui sont destinés à modifier l'état physique du courant de liquide (4) dans le voisinage de la surface (16) du capteur de détection (12), d'une manière telle qu'une précipitation ou un dépôt de la substance ou des substances que l'on doit détecter (9) sur la surface (16) du capteur de détection (12) peut être mis en oeuvre ou accéléré, c'est-à-dire en présence de cette substance ou de ces substances particulières (9) dans le courant de liquide (4) ; dans lequel un paramètre de détection (Td) qui est influencée par la présence d'une matière précipitée (20) sur la surface (16) peut être mesuré avec le capteur de détection (12) ; et dans lequel le composant (1) est muni d'un moyen d'alarme (23) destiné à générer une alarme lorsqu'un certain degré de modification du paramètre de détection concerné (Td) est altéré ou dépassé dans le but de signaler le fait que la présence de la substance ou des substances concernées (9) a été détectée, **caractérisé en ce que** le composant comprend en outre un capteur de référence (13) qui possède une surface (16) qui peut être mis en contact avec le courant de liquide (4) ; dans lequel les moyens de précipitation (19) qui ont été mentionné ci-dessus ne sont pas prévus pour modifier l'état physique du courant de liquide (4) dans le voisinage de la surface du capteur de référence (13) ; et dans lequel un paramètre de détection (Tr) qui n'est pas influencé par la présence d'une matière de précipitation sur le capteur de détection (12) peut être mesuré avec le capteur de référence (13) ; et dans lequel le composant (1) est muni d'un circuit de comparaison (26) destiné à comparer les mesures du paramètre de détection concerné (Td), qui émanent du capteur de détection (12) aux mesures (Tr) qui émanent du capteur de référence (13) dans le but de générer un signal d'entrée pour le moyen d'alarme (23).

2. Composant (1) selon la revendication 1, **caractérisé en ce que** les substances (9) qui doivent être détectées représentent des ions calcium dissous ; et **en ce que** le courant de liquide (4) est un courant d'eau.

3. Composant (1) selon une ou plusieurs des revendications précédentes, **caractérisé en ce que**, dans le but d'obtenir les moyens de précipitation (19), on prévoit des moyens avec lesquels on peut générer un champ électrique et/ou magnétique dans le courant de liquide (4).

4. Composant (1) selon une ou plusieurs des revendications précédentes, **caractérisé en ce que**, dans le but d'obtenir les moyens de précipitation (19), on prévoit un élément de chauffage (17) dans le capteur de détection (12), qui est conçu pour chauffer localement le courant de liquide (4) autour du capteur de détection (12).

5. Composant (1) selon une ou plusieurs des revendications précédentes, **caractérisé en ce que** le capteur de détection (12) et le capteur de référence (13) représentent des capteurs de température résistifs, de manière plus spécifique des capteurs de températures qui sont désignés par le terme « capteurs de température Pt100 » qui désigne un capteur de résistance à base de platine qui possède une résistance 100 ohms ; et dans lequel les paramètres de détection (Td, Tr) que l'on a mentionné ci-dessus représente la température interne qui règne dans le capteur de détection (12) et dans le capteur de référence (13) concernés.

6. Composant (1) selon une ou plusieurs des revendications précédentes, **caractérisé en ce que** le capteur de détection (12) représente un capteur de température Pt100 ; dans lequel ce capteur de température fait lui-même office d'élément de chauffage (17) pour chauffer le courant de liquide (4) dans le voisinage de la surface (16) du capteur de détection (12).

7. Composant (1) selon les revendications 1 et 5, **caractérisé en ce que** le circuit de comparaison (26) contient également un générateur d'impulsions (33) avec lequel on peut appliquer une impulsion de tension électrique (31) sur le capteur de référence (13) de même que sur le capteur de détection (12) ; et dans lequel on mesure, dans les deux capteurs (12, 13) par l'intermédiaire du circuit de comparaison (26), l'évolution des températures internes (Td, Tr), au cours de toute la durée de l'impulsion de tension (31) ou au cours de phénomènes de transition qui y sont associés ; et dans lequel la différence (ΔV) sert de base pour la génération d'un signal d'entrée pour le moyen d'alarme (23).

8. Composant (1) selon une ou plusieurs des revendications précédentes, **caractérisé en ce que** le composant (1) est réalisé sous la forme d'une gaine en laiton ou en matière plastique (2) qui comprend un filet de vis externe (25) qui peut être vissé dans un trou qui possède un filet de vis interne d'un conduit.

9. Procédé destiné à détecter la présence d'un ou de plusieurs éléments dissous (9) dans un courant de liquide (4), dans lequel il consiste à :
- procurer un capteur de détection (12) dans le courant de liquide (4) ;
- modifier l'état physique du courant de liquide (4) autour du capteur de détection (12) dans le but de mettre en oeuvre ou d'accélérer une précipitation ou un dépôt de la substance ou des substances à détecter (9) sur le capteur de détection (12), à condition que la substance ou les substances concernée(s) (9) soi(en)t présente(s) dans le courant de liquide (4) ;
- mesurer un paramètre de détection (Td) avec le capteur de détection (12), le paramètre en question étant influencé par la présence d'une matière précipitée (20) sur le capteur de détection (12) ; et
- lorsqu'un certain degré de modification des paramètres de détection concernée (Td) est altéré ou dépassé, générer une alarme dans le but de signaler le fait que la présence de la substance ou des substances concernée(s) (9) a été détectée, **caractérisé en ce qu'**il est fait usage d'un capteur de référence ouverte (13) dans le courant de liquide (4), qui est configuré pour détecter un paramètre de détection (Tr) qui n'est pas influencé par la présence d'une matière précipitée sur le capteur de détection (12) ;
dans lequel, dans le voisinage du capteur de détection (12), un état physique est modifié dans le but de mettre en oeuvre d'accélérer une précipitation ou un dépôt de la substance ou des substances à détecter (9) sur le capteur de détection (12), mais non sur le capteur de référence (13) ; et dans lequel, dans le but de détecter la présence de la substance ou des substances (9) dans le courant de liquide (4), le paramètre de détection concerné (Td, Tr) est mesuré dans les deux capteurs (12, 13) et ces mesures (Vr, Vd) sont comparées l'une à l'autre.

10. Procédé selon la revendication 9, **caractérisé en ce que** les substances (9) qui doivent être détectées dans le courant de liquide (4) représentent des ions calcium et/ou des ions magnésium dissous ; et **en ce que** le courant de liquide (4) est un courant d'eau.

11. Procédé selon la revendication 9 ou 10, **caractérisé en ce que** l'état physique qui est modifié autour du capteur de détection (12) représente une élévation de la température du courant de liquide (4) dans le voisinage du capteur de détection (12).

12. Procédé selon une ou plusieurs des revendications 9 à 11, **caractérisé en ce que** l'état physique qui est modifié autour du capteur de détection (12) représente un champ magnétique ou électrique modifié.

13. Procédé selon une ou plusieurs des revendications 9 à 12, **caractérisé en ce que** le paramètre de détection (Td) qui est mesuré par le capteur de détection (12) dans le but de détecter la présence de la substance ou des substances (9) représente le transfert de chaleur à travers le capteur de détection (12) ou la température interne (Td) régnant dans ce dernier.
